# EUROPEAN PATENT APPLICATION

(11) **EP 3 372 224 A1**
(43) Date of publication of application: **12.09.2018**
(21) Application number: 17000334.7
(22) Date of filing: 07.03.2017
(51) Int. Cl.: A61K 9/16, A61K 9/50, A61K 9/51

(54) **NEW CONTROLLED DRUG DELIVERY SYSTEM USING WATER MISCIBLE SOLVENTS FOR PRODUCTION OF DRUG LOADED MICRO- AND NANOPARTICLES**

(71) Applicant: Alrise Biosystems GmbH, 13125 Berlin (DE)
(72) Inventor: Albayrak, Celal, 13125 Berlin (DE)
(74) Representative: Jungblut & Seuss

(57) **Abstract**

The current application describes a process for the manufacture of drug-loaded micro-or nanoparticles carried out in water miscible solvents as well as drug-loaded micro- or nanoparticles manufactured according to the described process.

## Description

Drug delivery systems have in general great influence on the therapeutic success of treatments. They can improve the performance of drug by increasing the therapeutic efficacy and reducing the toxicity profile (J Am Chem Soc 2016,138 704) and increase patient adherence. Therefore the development of suitable drug delivery systems is building the main focus of the pharmaceutical research.

There are a lot of technologies that have been discussed and patented. Up to now all the methods used solvents for dissolving of polymers for manufacturing of dug loaded microspheres are either not water miscible solvents (see e.g. WO 2005/110425, WO 2013/083041) or partially water miscible solvents (see e.g. US 6899898)

Surprisingly we found out that a new method for the manufacturing of sustained release systems using water miscible solvents can be designed. The new technology is able to produce drug loaded nano-and microspheres.

Using only water miscible solvents for dissolving polymers can be used to produce drug loaded microspheres, which have many advantages like accelerating the formation process for Nano- and Microspheres. The acceleration of the production process has influence on the amount of initial burst and encapsulation efficiency (ChemEngTechnol 2012, 3, 618).

Especially, proteins and peptides are building blocks of life and are very promising examples of therapeutics. On the other hand, they have very short biological half-life and have very low oral bioavailability. Therefore they are often not available for enteral application. For chronical therapy proteins and peptides often need sophisticated formulations.

One possibility to solve the above problems is to develop injectable depot formulations. Injectable depot formulations are suitable for the treatment of chronicle and mental diseases.

Especially, the activity of enzymes in organic solvents is increased (Nature 2003, 49,241, or Catalysts 2016, 6, 32) and also the stability of peptide drugs in organic solvents (Int J Pharm 2008, 351, 1, WO 98/90152, US 2005/0009739).

The solvents have very sensitive influence on the transport properties (Release behaviour) of the membranes (Marcel Mulder, Basic Principles of Membrane Technology, 1996) and kinetics of solidification of the membrane.

Surprisingly we found out that fully water miscible solvents can also be used to dissolve the polymers for the production of nano- and micro-particles. Therefore, we have been able to develop a new process for the encapsulation of drugs in micro- or nanoparticles. According to the new process even sensitive drug compounds and/or drug compounds having low solubility can be encapsulated.

One aspect of the current invention is therefore a process for the manufacture of drug-loaded micro-or nanoparticles comprising the following steps:
a) preparing a solution of a polymer selected from polylactide, polyglycolide, and polyester copolymers comprising copolymerized units of lactic acid and/or glycolic acid in one or more water miscible solvents,
b) adding a solution of one or more drug compounds solved in one or more water miscible solvents
c) stirring
d) formation of micro- or nanoparticles by adding a solution containing cationic-, anionic-, and non-ionic surfactants under stirring condition,
   the added solution optionally further containing a buffer solution, one or more agents for adjusting the viscosity of the aqueous surfactant solution, and/or an agent for adjusting the ion strength of the solution,
e) stirring
f) elimination of solvents
g) collection of formed nano- or microparticles
h) optionally washing with water
i) optionally addition of polyvinylpyrolodine derivate or sugar, or sugar ester,
j) optionally lyophylization of the formed nano- or microparticles.

Another aspect of the current invention is the drug containing micro- and nanoparticles obtained by the process described herein.

The nature of biological active drugs to be encapsulated in nano- and microparticles according to the invention can be either hydrophilic or hydrophobic. For the purpose of the present invention the terms "nano- and microparticles" are supposed to have the same meaning as the terms "nano- and microspheres".

The list of drugs that can be encapsulated in micro- or nanoparticles using the process according to the present invention is almost unlimited and includes peptides, proteins as well as small molecules, e.g. Amlodipine Besylate, Anastrozole, Atorvastatin-Calcium, Candesartan cilexetil, Donepezil HCl, Enalapril maleate, Paclitaxel, Docetaxel Anhydrous, Exenatide, Goserelin acetate, Triptorelin Acetate, Leuprolide acetate, degarelix acetate, Insulin, Octreotide Acetate, Olmesartanmedoxomil, Raloxifene HCl, Rosuvastatin calcium, Telmisartan, Tolterodine tartrate, Simvastatin, just to name a few.

Drugs should be just dissolved in following solvents or mixtures of them:

| Solubility in Water of various Solvents | (%) |
|---|---|
| Dimethylsulfoxide (DMSO) ............................. | 100.0 (Fully Miscible) |
| N-Methyl-2-pyrrolidone (NMP) ....................... | 100.0 (Fully Miscible) |
| Tetrahydrofurane (THF) ................................ | 100.0 (Fully Miscible) |
| Acetone............................................................ | 100.0 (Fully Miscible) |
| Methylethylketon.............................................. | 27.3 (Partially Miscible) |
| Methyl acetate ................................................ | 23.8 (Partially Miscible) |
| Ethyl Formiate ................................................ | 8.2 (Partially Miscible) |
| Ethyl Acetate .................................................. | 7.7 (Partially Miscible) |

The solvents listed in the table above are the most preferred solvents for the process according to the present invention. They can be used individually or in mixtures.

The nano- and/or microparticles provided by the process in accordance with the invention contain a therapeutically active agent embedded in a polymer matrix or encapsulated by a polymer shell, and said polymer matrix or polymer shell comprises a polymer selected from polylactide, polyglycolide, and polyester copolymers comprising copolymerized units of lactic acid and/or glycolic acid. Such polymers are advantageously biodegradable, and thus suitable e.g. for parenteral injection. As will be understood, the reference to (co-)polymerized units of lactic acid and/or glycolic acid herein refers to the units as they are contained in the polymer, with bonds formed to adjacent units.

However, (co)polymers of lactic acid and/or glycolic acid are typically obtained using lactide and/or glycolide as starting materials, and the (co) polymers are thus frequently referred to as "polyglycolide", "polylactide", "copolymer of lactide" with a given comonomer, or "copolymer of glycolide" with a given comonomer.

In the context of the present application, (co)polymers obtained from other forms of polymerization starting from lactic acid and/or glycolic acid is not excluded by this terminology.

Such polymers are known to the skilled person and established for use in the medical field (e.g. Biodegradable polymers in clinical use and clinical Development, Edited by A. Domb, N. Kumar and A. Ezra, Wiley,2011, Long Acting Injections and Implants Editors: J.C. Wright and D.J. Burgess, Springer 2012). Suitable polymers include polyglycolide homopolymers (PGA, also referred to as polyglycolic acid), and polylactide homopolymers (PLA, also referred to as polylactic acid). Since polymers formed from lactic acid as monomer units can contain the units in D- or L-configuration, lactic acid may form a homopolymer containing only one of the two enantiomers (e.g. poly(L- lactic acid), PLLA), or a polymer combining units of L- and D- lactic acid. The latter are also referred to as stereo-copolymers. The stereo-copolymers may have different arrangements of the comonomers, and form e.g. random or block copolymers (e.g. poly(DL- lactic acid) random copolymer, or L- lactic acid /DL- lactic acid copolymers. Such stereo-copolymers formed from lactic acid as monomer unit are also suitable for use in the context of the invention. Unless indicated otherwise in a specific context, reference to polymerized lactic acid or lactide units includes L- lactic acid units, D-lactic acid units, or combinations of the two. Moreover, unless indicated otherwise in a specific context, reference to polylactide homopolymers includes not only polymers consisting of D- lactic acid or L- lactic acid units, but also polymers combining D- lactic acid and L- lactic acid units.

As particularly suitable polyester copolymers comprising copolymerized units of lactic acid and/or glycolic acid, reference can be made to a copolymer of lactide and glycolide, i.e. poly(lactide-co-glycolide), also referred to as poly(lactic-co-glycolic acid) or as PLGA. As will be appreciated by the skilled person, the degradation rate of such polymers after administration can be controlled by the ratio of copolymerized units of lactic acid to glycolic acid in the copolymer. Among these poly(lactide-co-glycolide) copolymers, preferred are those wherein the content of polymerized lactic acid units is at least 50 mol%, and in particular those wherein the content of polymerized lactic acid units is 50 to 85 mol%, such as 50 or 75 mol%, based on the total amount of polymerized units. As examples of other suitable comonomers that may be present in polyester copolymers comprising copolymerized units of lactic acid and/or glycolic acid, one or more comonomers selected from tetramethylglycolide, y-valerolactone, ε-caprolactone, trimethylene carbonate, tetramethylglycolide, and ethylene glycol may be mentioned. Thus, exemplary polyester copolymers, preferably binary copolymers, comprising copolymerized units of lactic acid and/or glycolic acid include a copolymer selected from the group consisting of a copolymer of glycolide and tetramethylglycolide, a copolymer of glycolide and -valerolactone, a copolymer of glycolide and ε-caprolactone, a copolymer of glycolide and trimethylene carbonate, a copolymer of lactide and tetramethylglycolide, a copolymer of lactide and -valerolactone, a copolymer of lactide and ε-caprolactone, a copolymer of lactide and trimethylene carbonate, a copolymer of glycolide and ethylene glycol, and a copolymer of lactide and ethylene glycol.

The polyester copolymers include random copolymers, block copolymers and gradient copolymers. Suitable block copolymer architectures include, e.g. AB block copolymers (e.g. AB block polymers comprising a polylactide (PLA) block and a poly(ethylene glycol) (PEG) block), ABA tri-block copolymers (e.g. ABA tri-block copolymers comprising PLA-PEG-PLA), star-shaped block copolymers (e.g. S(3)-PEG-PLA block copolymers and S(4)-PEG-PLA block copolymers).

In the polyester copolymers comprising copolymerized units of lactic acid and/or glycolic acid for use in the context of the present invention, it is preferred that the amount of the copolymerized units of lactic acid and/or glycolic acid (i.e. the amount of copolymerized units of lactic acid, if no glycolic acid is copolymerized, the amount of copolymerized units of glycolic acid, if no lactic acid is copolymerized, or the sum of the amounts of copolymerized units of glycolic acid and lactic acid, if both are copolymerized) accounts for at least 50 mol% of all copolymerized units in the copolymer. It is more preferred that that the amount of the copolymerized units of lactic acid and/or glycolic acid accounts for at least 70 mol% of all copolymerized units in the copolymer.

It will be appreciated by the skilled reader that the degradation rate of the nano- and/or microparticles of the invention can be influenced by the molecular weight of the polymer. Polymers of different molecular weights (or inherent viscosities) can be mixed to yield a desired degradation profile. Generally, polymers with an intrinsic viscosity in the range of 0.1 to 3 dL/g, preferably to 2 dL/g (0.1 % (w/v), chloroform, at 25°C) are used.

Particularly preferred for use in the context of the present invention are nano- and/or microparticles comprising a poly(lactide-co-glycolide) copolymer, i.e. a copolymer consisting of glycolic acid and lactic acid units. In terms of their relative amount of glycolic acid and lactic acid units, preferred are those wherein the content of polymerized lactic acid units is at least 50 mol%, and in particular those wherein the content of polymerized lactic acid units is 50 to 85 mol%, such as 50 or 75 mol%, based on the total amount of polymerized units. Blends of poly(lactide-co-glycolide) copolymers with different relative amounts of glycolic acid and lactic acid, favourably within the above preferred limits, may also be used.

Suitable commercially obtainable polymers for use in the nano- and/or microparticles according to the present invention include, but are not limited to Resomer® (EVONIK) L-104, L-206, L-207, L-208, L-209, L-210, L-214, R-104, R-202, R-203, R-206, R-207, R-208, G-110, G-205, LR-909, RG-502, RG-502H, RG-503, RG-503H, RG-504, RG 504H, RG-505, RG-505H, RG-506, RG-508, RG-752, RG-752H, RG-753, RG753H, RG-755, RG-756, RG-757 and Resomer® RG-858.

The polymer matrix or polymer shell of the nano- and/or microparticles may comprise one type of polymer selected from polylactide, polyglycolide, and polyester copolymers comprising copolymerized units of lactic acid and/or glycolic acid, or two or more types of polymers selected from polylactide, polyglycolide, and polyester copolymers comprising copolymerized units of lactic acid and/or glycolic acid, e.g. as a polymer blend. If two or more types are used, the polymers may differ e.g. in the type of polymerized monomer units, or in the relative ratios thereof. It is preferred that the one or more polymers selected from polylactide, polyglycolide, and polyester copolymers comprising copolymerized units of lactic acid and/or glycolic acid account for 70 wt.% or more, in particular 80 wt.% or more of the polymers in the polymer matrix or polymer shell of the nano- and/or microparticles. It is particularly preferred that the polymer matrix or polymer shell does not contain any other polymer component, apart from the one or more polymers selected from polylactide, polyglycolide, and polyester copolymers comprising copolymerized units of lactic acid and/or glycolic acid.

The concentration of the surfactant in the aqueous surfactant solution is preferably in the range of 0.1% (w/v) to 30% (w/v), preferably 0.1% to 20%, and more preferably 0.1 to 5%, based on the total volume of the surfactant solution. As will be understood by the skilled reader, the concentration in weight by volume corresponds to the amount of the solute in g per 100 ml of the total volume of the solution including the surfactant, typically at 20°C.

Surfactants suitable for the aqueous surfactant solution encompass cationic-, anionic-, and non-ionic surfactants. Exemplary surfactants can be selected from polyoxyethylene-polyoxypropylene block copolymers, in particular polyoxyethylene-polyoxypropylene-polyoxyethylene-triblock copolymers such as Poloxamer®, Poloxamine®, polyethylene glycol alkyl ethers, fatty acid esters of polyoxyethylensorbitan, especially polyoxyethylenesorbitanmonooleate and polyoxyethylenesorbitanmonolaurate also referred to as polysorbates (Tween®, Span®), sucrose esters (Sisterna®, Ryoto Sugar Ester, Tokyo), gelatin, polyvinylpyrrolidone, fatty alcohol polyglycosides, Charps, Charpso, decyl-ß-D-glycopyranoside, decyl-ß-D-maltopyranoside, dodecyl-ß-D-maltopyranoside, sodium oleate, polyethylene glycol, polyvinyl alcohol (PVA), polyethoxylated fatty acid ethers (Brij®), Triton X 100 or mixtures thereof. Preferred as a surfactant are polyvinyl alcohol, polyoxyethylene-polyoxypropylene-polyoxyethylene-triblock copolymers and fatty acid esters of polyoxyethylensorbitan, especially polyoxyethylenesorbitanmonooleate and polyoxyethylenesorbitanmonolaurate, or mixtures thereof.

The term "stirring" as mentioned in steps c) and e) of the process described above refers to a high-shear agitation such as achieved by use of a mechanical agitator (e.g. DispermatCA, Getzmann GmbH) equipped with a 2.0 cm dissolver disc for 6 minutes at 2000-5000 rpm.

The aqueous surfactant solution may contain other components besides the water, optional cosolvents and the surfactant, e.g. a buffer (such as acetate buffer, phosphate buffer, citrate buffer, TRIS or, HEPES-buffer), or an agent for adjusting the viscosity of the aqueous surfactant solution, or an agent for adjusting the ion strength of the solution. For example, the aqueous surfactant solution may comprise a dissolved physiologically tolerable salt, such as NaCl, MgCl₂, CaCl₂ or dissolved sugar (such as glucose, fructose, saccharose or dextran).

A major advantage of the process according to the invention is that it can be carried out at room temperature, so that sensitive drug compounds will not be altered by exposure to enhanced temperature. Depending on the drug compounds, the polymers and further ingredients the process may also be carried out at different temperatures, e.g. between -5°C and 40°C, preferably between 5°C and 35°C.

### EXAMPLES

The current invention is further illustrated by the following examples which are not limiting the invention in any way.

### Example 1:

1.5 g polymer of RG 756 is dissolved in 6 mL solvent (composed of 80% THF and 20% acetone) and transferred to a glass vessel (inner height 11.0 cm, inner diameter 2.7 cm).

50 mg Octreotide is dissolved in 0.7 mL DMSO and added to the polymer solution and then stirred by means of a mechanical agitator (DispermatCA, Getzmann GmbH) equipped with a 2.0 cm dissolver disc for 6 minutes at 2500 rpm at room temperature.

40 mL acetate buffer containing 3% poloxamer 188 and 10% NaCl is added under continous stirring as described above by means of a peristaltic Pump (Ecoline, Ismatec) equipped with a silicon tube (inner diameter 2 mm) at a rate of 140 mL/min.

The microsphere suspension is transferred to a two-necked round bottom flask and agitated with a magnetic stirrer.

The organic solvents THF and acetone are eliminated at room temperature by applying vacuum or extraction.

The drug containing microspheres are collected by filtration and washed with water.

The solvent-and almost surfactant-free microsphere suspensions are lyophilized.

If it is desired, for example polyvinylpyrolodine derivate or sugar, or sugar ester can be added to the microparticle suspension before lyophilization.

The amount of drugs containing in micro particles is determined using HPLC- method.

The microparticles contain 1.23 % drugs and the encapsulations efficiency is about 75%

### Examples 2 :

1.5 g polymer of RG 756 is dissolved in 5 mL solvent (composed of 80% THF and 20% acetone) and transferred to a glass vessel (inner height 11.0 cm, inner diameter 2.7 cm).

130 mg Anastrazole is dissolved in 0.5 ml solvent composed of 80%THA and 20 % aceton and added to the polymer solution and then stirred by means of a mechanical agitator (Dispermat CA, Getzmann GmbH) equipped with a 2.0 cm dissolver disc for 8 minutes at 3000 rpm at room temperature.

40 mL acetate buffer containing 3% poloxamer 188 and 10% NaCl is added by means of a peristaltic Pump (Ecoline, Ismatec) equipped with a silicon tube (inner diameter 2 mm) at a rate of 140 mL/min.

The microspheres suspension is transferred to a two-necked round bottom flask and agitated with a magnetic stirrer.

The organic solvents THF and acetone are eliminated at room temperature by applying vacuum or extraction.

The drug containing microspheres are collected by filtration and washed with water.

The solvent- and almost surfactant-free microspheres suspensions are lyophilized.

If it is desired, for example polyvinylpyrolodine derivate or sugar, or sugar ester can be added to the microparticle suspension before lyophilization.

The amount of drugs containing in microspheres was determined using HPLC- method.

The microparticles contain 3.60 % drugs and the encapsulations efficiency was calculated and was 86.75%

### Example 3:

1.5 g polymer of RG 756 is dissolved in 6 mL solvent (composed of 80% THF and 20% acetone) and transferred to a glass vessel (inner height 11.0 cm, inner diameter 2.7 cm).

150 mg olmesartan-medoxomil is dissolved in 0.7 mL DMSO and added to the polymer solution and then stirred by means of a mechanical agitator (Dispermat CA, Getzmann GmbH) equipped with a 2.0 cm dissolver disc for 8 minutes at 3000 rpm at room temperature.

40 mL acetate buffer containing 3% poloxamer 188 and 10% NaCl is added by means of a peristaltic Pump (Ecoline, Ismatec) equipped with a silicon tube (inner diameter 2 mm) at a rate of 140 mL/min.

The microparticle suspension is transferred to a two-necked round bottom flask and agitated with a magnetic stirrer.

The organic solvents THF and acetone are eliminated at room temperature by applying vacuum or extraction.

The drug containing microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization.

The amount of drugs containing in microparticles was determined using HPLC- method.

The microparticles contain 3.33% drugs and the encapsulations efficiency was calculated and was about 70%

### Example 4:

1.5 g polymer of RG 756 is dissolved in 6 mL solvent (composed of 80% THF and 20% acetone) and transferred to a glass vessel (inner height 11.0 cm, inner diameter 2.7 cm).

50 mg pravastatin is dissolved in 0.5 mL DMSO and added to the polymer solution and then stirred by means of a mechanical agitator (Dispermat CA, Getzmann GmbH) equipped with a 2.0 cm dissolver disc for 8 minutes at 3000 rpm at room temperature.

40 mL acetate buffer containing 3% poloxamer 188 and 10% NaCl was added by means of a peristaltic Pump (Ecoline, Ismatec) equipped with a silicon tube (inner diameter 2 mm) at a rate of 140 mL/min.

The microparticle suspension is transferred to a two-necked round bottom flask and agitated with a magnetic stirrer.

The organic solvents THF and acetone are eliminated at room temperature by applying vacuum or extraction.

The drug containing microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lypohilization.

The amount of drugs containing in microparticles was determined using HPLC- method.

The microparticles contain 1.32 % drugs and the encapsulations efficiency was calculated and was about 80%.

### Example 5:

1.5 g polymer of RG 756 is dissolved in 6 mL solvent (composed of 80% THF and 20% acetone) and transferred to a glass vessel (inner height 11.0 cm, inner diameter 2.7 cm).

50 mg candesartan cilexetil is dissolved in 0.5 mL DMSO and added to the polymer solution and then stirred by means of a mechanical agitator (Dispermat CA, Getzmann GmbH) equipped with a 2.0 cm dissolver disc for 6 minutes at 2500 rpm at room temperature.

40 mL acetate buffer containing 3% poloxamer 188 and 10% NaCl is added by means of a peristaltic Pump (Ecoline, Ismatec) equipped with a silicon tube (inner diameter 2 mm) at a rate of 140 mL/min.

The microparticle suspension is transferred to a two-necked round bottom flask and agitated with a magnetic stirrer.

The organic solvents THF and acetone are eliminated at room temperature by applying vacuum or extraction.

The drug containing microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization.

The amount of drugs containing in microparticles determined using HPLC- method.

The microparticles contain 1.32 % drugs and the encapsulations efficiency was calculated and was about 80%

### Example 6

1.5 g polymer of RG 756 is dissolved in 6 mL solvent (composed of 80% THF and 20% acetone) and transferred to a glass vessel (inner height 11.0 cm, inner diameter 2.7 cm).

40 mg pramipexol is dissolved in 0.75 mL DMSO and added to the polymer solution and then stirred by means of a mechanical agitator (Dispermat CA, Getzmann GmbH) equipped with a 2.0 cm dissolver disc for 8 minutes at 3000 rpm at room temperature.

40 mL acetate buffer containing 3% poloxamer 188 and 10% NaCl is added by means of a peristaltic Pump (Ecoline, Ismatec) equipped with a silicon tube (inner diameter 2 mm) at a rate of 140 mL/min.

The microparticle suspension was transferred to a two-necked round bottom flask and agitated with a magnetic stirrer.

The organic solvents THF and acetone are eliminated at room temperature by applying vacuum or extraction.

The drug containing microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization.

The amount of drugs containing in microparticles was determined using HPLC- method.

The microparticles contain 1.23 % drugs and the encapsulations efficiency was about 75%

### Example 7:

1.5 g polymer of RG 756 is dissolved in 6 mL solvent (composed of 80% THF and 20% acetone) and transferred to a glass vessel (inner height 11.0 cm, inner diameter 2.7 cm).

75 mg carveldilol is dissolved in 0.5 mL DMSO and added to the polymer solution and then stirred by means of a mechanical agitator (Dispermat CA, Getzmann GmbH) equipped with a 2.0 cm dissolver disc for 10 minutes at 3000 rpm at room temperature.

40 mL acetate buffer containing 3% poloxamer 188 and 10% NaCl is added by means of a peristaltic Pump (Ecoline, Ismatec) equipped with a silicon tube (inner diameter 2 mm) at a rate of 140 mL/min.

The microparticle suspension was transferred to a two-necked round bottom flask and agitated with a magnetic stirrer.

The organic solvents THF and acetone are eliminated at room temperature by applying vacuum or extraction.

The drug containing microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization.

The amount of drugs containing in microparticles has been determined using HPLC- method.

The microparticles contain 2.2% drugs and the encapsulations efficiency was calculated and was about 91.67%

### Example 8:

1.5 g polymer of RG 756 is dissolved in 6 mL solvent (composed of 80% THF and 20% acetone) and transferred to a glass vessel (inner height 11.0 cm, inner diameter 2.7 cm).

150 mg Raloxifene HCL is dissolved in 0.5 mL DMSO and added to the polymer solution and then stirred by means of a mechanical agitator (Dispermat CA, Getzmann GmbH) equipped with a 2.0 cm dissolver disc for 10 minutes at 3000 rpm at room temperature.

40 mL acetate buffer containing 3% poloxamer 188 and 10% NaCl is added by means of a peristaltic Pump (Ecoline, Ismatec) equipped with a silicon tube (inner diameter 2 mm) at a rate of 140 mL/min.

The microparticle suspension is transferred to a two-necked round bottom flask and agitated with a magnetic stirrer.

The organic solvents THF and acetone are eliminated at room temperature by applying vacuum or extraction.

The drug containing microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization.

The amount of drugs containing in microparticles has been determined using HPLC- method.

The microparticles contain 6.97% drugs and the encapsulations efficiency was calculated and was about 78.7%.

### Example 9:

1.5 g polymer of RG 756 is dissolved in 6 mL solvent (composed of 78% Aceton and 22% Methylacetate) and transferred to a glass vessel (inner height 11.0 cm, inner diameter 2.7 cm)

The polymer solution stirred by means of a mechanical agitator (Dispermat CA, Getzmann GmbH) equipped with a 2.0 cm dissolver disc for 5 minutes at 3000 rpm at room temperature.

40 mL acetate buffer containing 3% poloxamer 188 and 20% NaCl is added by means of a peristaltic Pump (Ecoline, Ismatec) equipped with a silicon tube (inner diameter 2 mm) at a rate of 140 mL/min.

The microparticle suspension is transferred to a two-necked round bottom flask and agitated with a magnetic stirrer.

The organic solvents methyl acetate and acetone are eliminated at room temperature by applying vacuum or extraction.

The microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization.

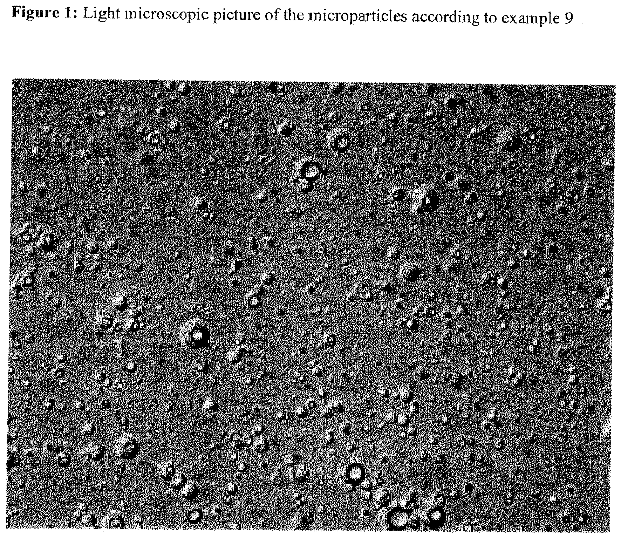

The light microscopic picture of the resulting microparticles is shown in figure 1.

### Example 10:

1.5 g polymer of RG 756 is dissolved in 6 mL solvent (composed of 70% Aceton and 30% Methyl ethyl keton) and transferred to a glass vessel (inner height 11.0 cm, inner diameter 2.7 cm)

The polymer solution is transferred to a vessel and then stirred by means of a mechanical agitator (Dispermat CA, Getzmann GmbH) equipped with a 2.0 cm dissolver disc for 6 minutes at 2500 rpm at room temperature.

40 mL acetate buffer containing 3%poloxamer 188 and 20% NaCl is added by means of a peristaltic Pump (Ecoline, Ismatec) equipped with a silicon tube (inner diameter 2 mm) at a rate of 140 mL/min.

The microparticle suspension is transferred to a two-necked round bottom flask and agitated with a magnetic stirrer.

The organic solvents methyl acetate and acetone are eliminated at room temperature by applying vacuum or extraction.

The microparticles are collected by filtration and washed with water.

The solvent- and almost surfactant-free microparticle suspensions are lyophilized.

If it is desired, for example polyvinylpyrolodine derivate or sugar, or sugar ester can be added to the microparticle suspension before lyophilization

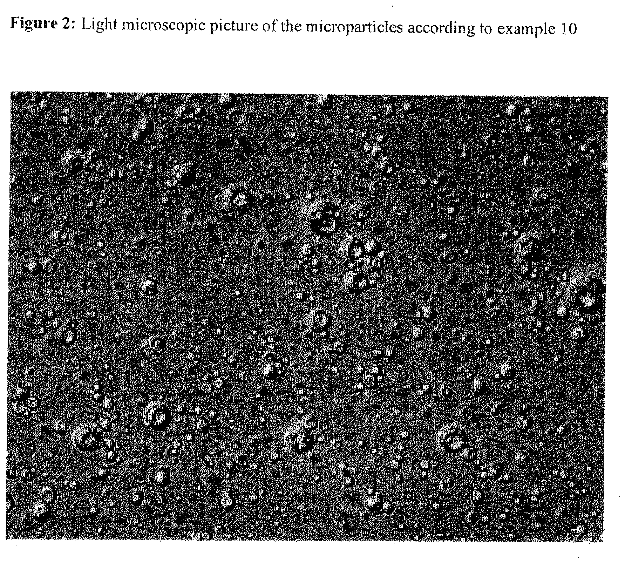

The light microscopic picture of the resulting microparticles is shown in figure 2.

## Claims

1. A process for the manufacture of drug-loaded micro-or nanoparticles comprising the following steps:
a) preparing a solution of a polymer selected from polylactide, polyglycolide, and polyester copolymers comprising copolymerized units of lactic acid and/or glycolic acid in one or more water miscible solvents,
b) adding a solution of one or more drug compounds solved in one or more water miscible solvents
c) stirring
d) formation of micro- or nanoparticles by adding a solution containing cationic-, anionic-, and non-ionic surfactants under stirring condition,
the added solution optionally further containing a buffer solution, one or more agents for adjusting the viscosity of the aqueous surfactant solution, and/or an agent for adjusting the ion strength of the solution,
e) stirring
f) elimination of solvents
g) collection of formed nano- or microparticles
h) optionally washing with water
i) optionally addition of polyvinylpyrolodine derivate or sugar, or sugar ester,
j) optionally lyophylization of the formed nano- or microparticles.

2. The process according to claim 1 in which the polymer is selected from poly(lactide-co-glycolide) copolymer, wherein the content of polymerized lactic acid units is at least 50 mol% based on the total amount of polymerized units
or
blends of poly(lactide-co-glycolide) copolymers with different relative amounts of glycolic acid and lactic acid, wherein the content of polymerized lactic acid units is at least 50 mol% based on the total amount of polymerized units.

3. The process according to claim 1 or 2 in which the drug compound is selected from Amlodipine Besylate, Anastrozole ,Atorvastatin-Calcium, Candesartan cilexetil, Donepezil HCl, Enalapril maleate , Paclitaxel, Docetaxel Anhydrous, Exenatide, Goserelin acetate, Triptorelin Acetate, Leuprolide acetate , degarelix acetate, Insulin, Octreotide Acetate, Olmesartanmedoxomil, Raloxifene HCl, Rosuvastatin calcium, Telmisartan, Tolterodine tartrate, Simvastatin.

4. The process according to at least one of claims 1-3 in which the water miscible solvent is selected from Dimethylsulfoxide (DMSO), N-Methyl-2-pyrrolidone (NMP), Tetrahydrofurane (THF), Acetone, Methylethylketone, Methyl acetate, Ethyl Formiate, Ethyl Acetate, or mixtures thereof.

5. The process according to at least one of claims 1-4 in which the surfactant is selected from polyoxyethylene-polyoxypropylene block copolymers, in particular polyoxyethylene-polyoxypropylene-polyoxyethylene-triblock copolymers, polyethylene glycol alkyl ethers, fatty acid esters of polyoxyethylensorbitan, especially polyoxyethylenesorbitanmonooleate and polyoxyethylenesorbitanmonolaurate, sucrose esters, gelatin, polyvinylpyrrolidone, fatty alcohol polyglycosides, Charps, Charpso, decyl-ß-D-glycopyranoside, decyl-ß-D-maltopyranoside, dodecyl-ß-D-maltopyranoside, sodium oleate, polyethylene glycol, polyvinyl alcohol (PVA), polyethoxylated fatty acid ethers, Triton X 100 or mixtures thereof.

6. The process according to at least one of claims 1-5 in which the surfactant containing solution further contains one or more buffers selected from, acetate buffer, phosphate buffer, citrate buffer, TRIS or HEPES-buffer or mixtures thereof.

7. The process according to at least one of claims 1-6 in which the surfactant containing solution further contains physiologically tolerable salt and/or a dissolved sugar.

8. The process according to at least one of claims 1-7 which is carried out at room temperature.

9. Micro- or nanoparticle obtained by the process of at least one of claims 1-8.
